# EUROPÄISCHE PATENTSCHRIFT

(11) **EP 2 554 132 B1**
(45) Veröffentlichungstag und Bekanntmachung des Hinweises auf die Patenterteilung: **02.03.2016**
(21) Anmeldenummer: 11176186.2
(22) Anmeldetag: 01.08.2011
(51) Int. Cl.: A61B 18/14

(54) **Gewebefusionsinstrument**
Tissue fusion instrument
Instrument de fusion des tissus

(43) Veröffentlichungstag der Anmeldung: 06.02.2013
(73) Patentinhaber: ERBE Elektromedizin GmbH, 72072 Tübingen (DE)
(72) Erfinder: Fischer, Klaus, 72202 Nagold (DE); Schäller, Daniel, 72070 Tübingen (DE); Brodbeck, Achim, 72555 Metzingen-Neuhaus (DE)
(74) Vertreter: Rüger, Barthelt & Abel

(56) Entgegenhaltungen:
- EP-A1- 1 348 391
- US-A1- 2005 010 211
- US-A1- 2009 112 202
- US-A1- 2009 248 007
- US-A1- 2010 100 122
- US-A1- 2011 028 971

## Beschreibung

Gewebefusionsinstrumente dienen beispielsweise zum Abklemmen und Schließen von Blutgefäßen durch Strom und Wärmewirkung. Zum Beispiel werden einander gegenüberliegende Gefäßwände durch zwei Elektroden aneinander gedrückt und durch Stromfluss so erwärmt, dass sie dauerhaft miteinander verkleben. Dabei soll die Wärmewirkung möglichst auf die zu fusionierenden Gewebepartien beschränkt bleiben. Es ist das Ziel, umliegendes Gewebe möglichst nicht zu beeinträchtigen oder zu schädigen.

Der Grundaufbau eines Gewebefusionsinstruments ist bspw. aus der US 2006/0264922 A1 und der den Oberbegriff des Patentanspruchs 1 offenbarenden US 2009/0248007 A1 zu entnehmen. Das Instrument ist in Form einer Zange mit zwei elektrisch gegeneinander isolierten Branchen ausgebildet. Zwischen den beiden Branchen der Zange kann ein Gefäß gefasst, abgeklemmt und durch Stromwirkung koaguliert werden.

Zur Vermeidung von Wärmeeintrag in umgebendes Gewebe schlägt die US 2010/0204698 A1 eine aktive Kühlung zweier beweglicher Elektroden vor, wobei zur aktiven Kühlung thermoelektrische Elemente dienen. Das heiße Ende des thermoelektrischen Elements ist wiederum durch eine Fluidkühlung gekühlt, um Wärme abzuführen.

Gewebefusionsinstrumente sollen möglichst leicht zu handhaben sein und schlanke Branchen aufweisen, damit auch unter beengten Platzverhältnissen ein sicheres und leichtes Arbeiten möglich ist.

Es ist Aufgabe der Erfindung, ein Gewebefusionsinstrument zu schaffen, das einfach aufgebaut ist und bei der Gewebefusion umliegendes Gewebe möglichst schont.

Diese Aufgabe wird mit dem Gewebefusionsinstrument nach Anspruch 1 gelöst.

Das erfindungsgemäße Gewebefusionsinstrument weist mindestens einen Elektrodenträger auf, der mit einer elektrischen Zuleitung verbunden ist oder Verbindungsmittel zum Anschluss einer elektrischen Zuleitung aufweist. Solche Anschlussmittel können Buchsen, Klemmen oder dergleichen sein.

Der Elektrodenträger ist vorzugsweise mechanisch steif und elektrisch leitend ausgebildet. Er bildet bspw. die mechanisch tragende Struktur einer Branche einer Gewebefusionszange. Die Steifigkeit des Elektrodenträgers wird durch eine geeignete Materialwahl (z.B. Stahl) und einen geeigneten Querschnitt des Elektrodenträgers sichergestellt. Es wird eine hohe Biegesteifigkeit angestrebt um die nötigen Schließkräfte einer Gewebefusionszange zu erbringen. Der Elektrodenträger weist dazu einen Querschnitt mit hohem Flächenträgheitsmoment auf, um ein hohes Biegewiderstandsmoment zu erhalten.

Der Elektrodenträger ist mit einer Elektrode versehen, die dazu dient, mit dem zu fusionierenden Gewebe direkt in Berührung gebracht zu werden. Die Elektrode ist mit dem Elektrodenträger elektrisch und mechanisch verbunden. Zur Verbindung dienen eine oder mehrere punktuelle Verbindungen. Es handelt sich bei diesen punktuellen Verbindungen um Stellen, an denen der Elektrodenträger, oder fest mit dem Elektronenträger verbundene Metallteile, mit der Elektrode stoffschlüssig, zum Beispiel durch Löten, Kleben oder Schweißen, verbunden ist oder sind. Bei der punktuellen Verbindung handelt es sich somit bspw. um Klebe-, Schweißpunkte, Folgen von Klebe-, Schweißpunkten oder Schweißnähte. Jedenfalls aber ist keine flächenhafte Verbindung zwischen dem Elektrodenträger und der Elektrode vorgesehen.

Der Elektrodenträger und die Elektrode definieren miteinander eine Trennfuge oder auch einen Spalt. Die Elektrode kann bei einigen Ausführungsformen flächig an dem Elektrodenträger anliegen, wobei jedoch die vorhandene Fuge den Wärmeübergang von der Elektrode zu dem Elektrodenträger behindert. Die lediglich punktuelle Verbindung in Form von Schweißpunkten oder Nähten überträgt wenig Wärme von der Elektrode auf den Elektrodenträger.

Die Elektrode kann als relativ dünne, ebene, profilierte oder auch gewölbte Platte ausgebildet sein, die mit einer oder mehreren Öffnungen versehen sein kann. Die Wärmkapazität dieser Elektrode bleibt relativ gering. Bei der Erwärmung von Gewebe erwärmt sich die Elektrode zunächst mit, wobei sie aber wegen ihres geringen Volumens und der damit verbundenen geringen Wärmekapazität nach Gebrauch relativ schnell wieder abkühlt. Die größere Wärmekapazität des Elektrodenträgers ist durch die lediglich punktuelle stoffliche Verbindung zwischen der Elektrode und dem Elektrodenträger von der Elektrode weitgehend entkoppelt, so dass sich der Elektrodenträger nur wenig erwärmt und somit auch wenig Wärme an die Elektrode zurückgeben kann. Somit wird bei der Gewebefusion ein gutes Fusionsergebnis sichergestellt, denn es wird vermieden, dass die Elektrode schon beim Fassen eines Gefäßes eine zu hohe Temperatur aufweist, die eine vorzeitige Eiweißdenaturierung bewirken könnte.

Weiter kann durch die weitgehende Unterbindung des Wärmeflusses von der Elektrode zum Elektrodenträger verhindert werden, dass der Elektrodenträger, z.B. an seiner Rückseite so warm wird, dass er umliegende Gewebe schädigt. Außerdem kann der Elektrodenträger mit einem Kunststoffüberzug versehen sein oder in einen Kunststoffkörper eingebettet sein. Die thermische Belastung des Kunststoffs wird durch die erfindungsgemäße Maßnahme reduziert. Außerdem wird durch den Kunststoff eine weitere thermische und elektrische Isolation bewirkt, die weiter dazu beiträgt, dass an der Elektrode entstehende Wärme von der Rückseite des Gewebefusionsinstruments bzw. des Elektrodenträgers ferngehalten wird.

Der zwischen Elektrode und Elektrodenträger vorhandene Spalt kann je nach Anwendungsfall eine unterschiedliche Weite aufweisen. Er kann z.B. Null (0) sein, d.h. die Elektrode und der Elektrodenträger liegen ohne erkennbaren Abstand einander an. Vorzugsweise kommen dann die Elektrode und der Elektrodenträger ohne mechanische Krafteinwirkung, also mechanisch spannungslos zur gemeinsamen Anlage. Der Abstand kann jedoch auch mehrere Zehntel Millimeter bis hin zu einem Millimeter groß, gegebenenfalls auch größer sein. Vorzugsweise wird der so vorhandene Abstand nur an wenigen Stellen von den punktuellen Verbindungen metallisch überbrückt. In dem Spalt kann ein gasförmiges Medium beispielsweise Luft oder auch ein thermisches Isoliermaterial wie Kunststoff, Keramik oder dergleichen angeordnet sein. Der Spalt kann nach außen geschlossen sein. Beispielsweise kann er von Kunststoff überbrückt sein. Dazu kann der Kunststoff dienen, mit dem der Elektrodenträger beschichtet oder in den der Elektrodenträger eingebettet ist.

Der Elektrodenträger und die Elektrode können aus unterschiedlichen Materialien, insbesondere unterschiedlichen Metallen ausgebildet sein. Zum Beispiel kann das Material der Elektrode auf eine geringe Wärmekapazität und ein hohes Wärmeleitvermögen hin ausgewählt sein, um an der Elektrode eine möglichst gleichmäßige Temperaturverteilung zu erreichen. Der Elektrodenträger kann bspw. nach mechanischen Gesichtspunkten im Hinblick auf eine hohe Steifigkeit ausgewählt sein, wobei sich dann irgendwelche anderen Werte für Wärmeleitfähigkeit und Wärmekapazität ergeben.

Zur Ausbildung eines Abstands zwischen der Elektrode und dem Elektrodenträger können die Elektrode und zusätzlich oder alternativ der Elektrodenträger mit einem oder mehreren Vorsprüngen versehen sein, an denen die entsprechenden stoffschlüssigen Verbindungspunkte, d.h. z.B. Schweißpunkte ausgebildet sind. Es ist jedoch auch möglich, anstelle dieser Vorsprünge oder ergänzend dazu Abstandselemente, z.B. in Form von einem oder mehreren zwischen die Elektrode und den Elektrodenträger zu legenden Elementen vorzusehen, an denen die Verbindungspunkte ausgebildet sind. Die Abstandshalter können aus stofflich beispielsweise Kleben, Löten, oder Schweißen verbindbaren metallischen oder nicht metallischen Werkstoffen oder einer Kombination dieser Werkstoffe gebildet sein. Die Verbindungspunkte können bspw. an stirnseitigen Enden von schmalen Steg- oder drahtartigen Zwischenelementen ausgebildet sein und dabei sowohl eine Verbindung zu der Elektrode wie auch eine Verbindung zu dem Elektrodenträger herstellen.

Es ist auf Basis der vorgestellten Möglichkeiten eine Vielzahl von Ausführungsformen möglich, bei denen die Elektrode und der Elektrodenträger ohne flächenhafte stoffliche metallische Verbindung auskommen oder ohne mechanische Krafteinwirkung gegen einander in Anlage kommen. Damit ist eine Wärmebarriere geschaffen, die den Wärmestrom von der Elektrode zu dem Instrumentenrücken behindert. Die punktuelle Verbindung zwischen der Elektrode und dem Elektrodenträger kann auch eine mittelbare Verbindung sein, beispielsweise indem zwischen der Elektrode und dem Elektrodenträger anstelle draht- oder stegartiger Abstandshalter flächige Abstandshalter angeordnet werden. Die metallischstoffliche Verbindung kann dann wiederum punktuell von der Elektrode zu dem Abstandshalter und vorzugsweise an anderer Stelle von dem Abstandshalter zu dem Elektrodenträger geschaffen werden. Es sind somit zwei oder mehrere Spalte zwischen der Elektrode und dem Elektrodenträger vorhanden, wobei hinsichtlich dieser Spalte die vorstehenden Erläuterungen entsprechend gelten.

Die Elektrode kann mit einer oder mehren Öffnungen versehen sein, die z.B. zur Aufnahme weiterer Elemente dienen. Solche Elemente können sogenannte Einleger z.B. aus Kunststoff oder Keramik sein, die das zu fusionierende Gefäß z.B. mit einer Rippe oder anderen Vorsprüngen festhalten. Auch können ein oder mehrere Abstandshalter vorgesehen sein, bspw. in Form kleiner Keramikstifte, die verhindern, dass sich die gegenüber liegenden Elektroden einer Gewebefusionszange ungewollt berühren.

Zwischen Elektrode und dem Elektrodenträger können Abstandshalter aus Metall oder auch aus anderen Materialien wie z.B. Kunststoff oder Keramik angeordnet sein. Diese Abstandshalter können auch Teil von Elementen sein, die sich durch die Elektrode hindurch erstrecken und weitere Funktionen erbringen, wie bspw. ein Festhalten des Gefäßes und/oder die Verhinderung direkter Elektrodenberührung gegenüberliegender Elektroden einer Gewebefusionszange.

Das erfindungsgemäße chirurgische Gewebefusionsinstrument kann eine Gewebefusionszange oder auch ein laproskopisches Instrument sein, das wenigstens ein bewegliches Werkzeug aufweist, das am Ende eines langen Schaftes angeordnet ist. Grundsätzlich und insbesondere bei solchen laproskopischen Instrumenten kann zusätzlich ein Schneidelement zum Durchtrennen des behandelten Gewebes vorhanden sein. Die Schneidfunktion kann durch ein oder mehrere bewegliche Messer erbracht werden, die vorzugsweise beweglich angeordnet sind. So kann mit einem beweglichen Messer, das zwischen zwei Elektroden gefasste koagulierte Gefäß vom Chirurgen bewusst durchtrennt werden.

In der Zeichnung sind Ausführungsbeispiele der Erfindung veranschaulicht. Es zeigen:
Figur 1 eine Gewebefusionszange in schematisierter Prinzipdarstellung,
Figur 2 ein laproskopisches Fusionsinstrument, in schematisierter Prinzipdarstellung,
Figur 3 ein Ausschnitt einer Branche der Fusionszange nach Figur 1 oder des Instruments nach Figur 2, in schematisierter vereinfachter Perspektivdarstellung,
Figur 4 bis 11 verschiedene Ausführungsformen von Branchen von Fusionszangen oder laproskopischen Instrumenten jeweils im Vertikalschnitt,
Figur 12 eine weitere Ausführungsform einer Branche, in vereinfachter Seitenansicht,
Figur 13 eine abgewandelte Ausführungsform einer Branche mit Abstandselement, in perspektivischer Explosionsdarstellung,
Figur 14 die Branche nach Figur 13, in zusammengebautem Zustand,
Figur 15 eine weitere Ausführungsform einer Branche, in geschnittener Perspektivdarstellung,
Figur 16 eine Branche mit keramischen Abstandselementen zwischen Elektrode und Elektrodenträger, in ausschnittsweise Perspektivansicht,
Figur 17 die Branche nach Figur 16, im Vertikalschnitt,
Figur 18 und 19 weitere Ausführungsformen einer Branche, im Vertikalschnitt.

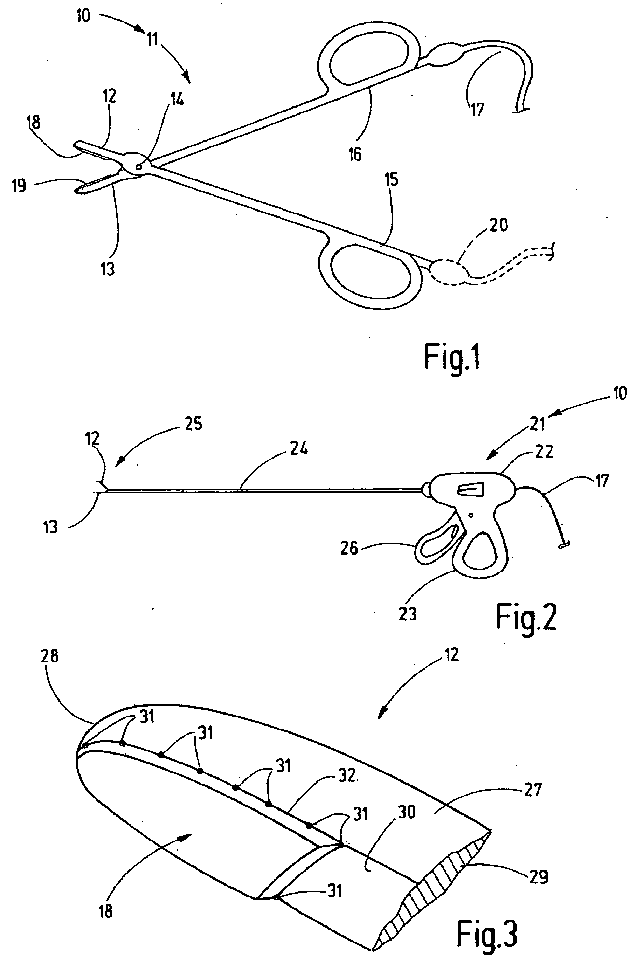

In Figur 1 ist ein elektrochirurgisches Gewebefusionsinstrument 10 in Gestalt einer Gewebefusionszange 11 veranschaulicht. Diese weist eine erste Branche 12 und eine zweite Branche 13 auf, die untereinander gelenkig verbunden sind. Dazu dient beispielsweise ein Scharniergelenk 14. Die Branchen 12, 13 sind mit Handgriffen 15, 16 verbunden, über die die Branchen 12, 13 aufeinander zu und voneinander weg bewegt werden können. Wenigstens einer der Handgriffe 15, 16 ist mit einer elektrischen Zuleitung 17 versehen, über die an den Branchen 12, 13 vorhandene Elektroden 18, 19 zur Koagulation eines zwischen den Branchen 12, 13 erfassten Gefäßes bestrombar sind. Alternativ zu einer Bauform mit einer einzigen (zweiadrigen) Zuleitung 17 an dem Handgriff 16 kann auch jeder der Handgriffe 15, 16 jeweils mit einer (dann z.B. einadrigen) elektrischen Zuleitung 17 bzw. 20 versehen sein.

Das Gewebefusionsinstrument 10 kann, wie Figur 2 schematisch zeigt, auch als laproskopisches Instrument 21 ausgebildet sein. Es weist ein mit einer elektrischen Zuleitung 17 versehenes Gehäuse 22 auf, das mit mindestens einem Griff 23 zur Handhabung durch den Arzt versehen ist. Von dem Gehäuse 22 erstreckt sich ein länglicher Schaft 24 weg an dessen Ende ein Werkzeug 25 zum Fassen und Koagulieren sowie gegebenenfalls Durchtrennen eines Gefäßes vorgesehen ist. Das Werkzeug 25 umfasst die beiden Branchen 12, 13 mit den hier nicht gesondert dargestellten Elektroden 18, 19, die wie bei Figur 1 an den einander zugewandten Seiten der Branchen 12, 13 angeordnet sind. Durch den Schaft 24 erstreckt sich ein Betätigungsmechanismus, der durch einen beweglichen Teil 26 des Griffs 23 betätigbar ist. Weitere Bedienelemente können an dem Teil 26 und/oder dem Gehäuse 22 sowie auch an dem Griff 23 vorgesehen sein.

Die nachfolgende Beschreibung der Branche 12 gilt entsprechend für die Branche 13. Dies gilt für alle Ausführungsformen der Branche 12, die nachstehend beschrieben sind. Diese Ausführungsformen können sowohl bei der Gewebefusionszange 11, wie auch bei dem laproskopischen Instrument 21 zur Anwendung kommen.

Die Branche 12 (Figur 3) weist einen Elektrodenträger 27 auf, der zum Beispiel aus einem massiven Metall (z.B. Stahl, vernickelter Stahl oder dergleichen) ausgebildet sein kann. Der Elektrodenträger 27 hat im vorliegenden Beispiel einen etwa rechteckigen oder quadratischen Querschnitt und verjüngt sich zu seinem freien Ende 28 hin. Er kann jedoch auch abweichende Querschnitte aufweisen. Insbesondere kann er an seiner von der Elektrode 18 abliegendem Rücken 29 gerundet sein. Unabhängig davon weist er an der dem Rücken 29 gegenüberliegenden Seite eine Fläche 30 zur Aufnahme der Elektrode 18 auf. Die Fläche 30 kann eine glatte, ununterbrochene oder auch profilierte, zum Beispiel genoppte, gewellte, geriffelte oder anderweitig profilierte Fläche sein, die gegebenenfalls auch eine oder mehrere Ausnehmungen, beispielsweise in Gestalt von Sackbohrungen aufweisen kann. An der Fläche 30 ist die Elektrode 18 angeordnet. Diese wird im einfachsten Fall durch eine aus Metall bestehende Platte gebildet. Sie kann flächenhaft an der Fläche 30 anliegen, so dass zwischen der Elektrode 18 und der Fläche 30 zwar ein Spalt 32, jedoch kein Abstand gebildet ist. Es kann jedoch auch ein kleiner oder größerer Abstand von zum Beispiel einigen Hundertstel oder Zehntel Millimetern vorgesehen sein. Der Abstand kann leer oder durch einen z.B. aus einem nichtmetallischen Material bestehenden Abstandshalter gefüllt sein.

Die Elektrode 18 ist mit der Branche 12 über punktuelle Verbindungen 31 verbunden, die beispielsweise durch Klebepunkte oder Schweißpunkte, beispielsweise Laserschweißpunkte gebildet sind. Es sind vorzugsweise mindestens zwei solcher Verbindungen 31 vorgesehen. Sind mehrere solcher Verbindungen 31 vorgesehen, erstrecken sie sich entlang des zwischen der Elektrode 18 und dem Elektrodenträger 27 gebildeten Spalts 32 in einer Reihe. Die Verbindungen 31 stellen eine stoffflüssige metallische Verbindung zwischen der Elektrode 18 und dem Elektrodenträger 27 her. Diese Verbindung dient der mechanischen Befestigung der Elektrode 18 an dem Elektrodenträger 27 und zur sicheren Stromübertragung zwischen beiden. Wegen des geringen Querschnitts der Verbindungen 31 kann jedoch über diese nur ein geringer Wärmefluss auftreten.

Vorzugsweise ist die der Fläche 30 zugewandte Rückseite der Elektrode 18 glänzend ausgebildet. Außerdem ist die Fläche 30 vorzugsweise glänzend ausgebildet. Auf diese Weise ist die durch Wärmestrahlung bedingte Wärmeübertragung über den Spalt 32 hinweg minimiert. Unabhängig von der Weite des Spalts 32 ist somit eine erste Barriere für die Wärmeübertragung gebildet. Diese Barriere wird auch nicht durch direkte Wärmeleitung überbrückt, wenn die Rückseite der Elektrode 18 nur lose, das heißt ohne mechanische Vorspannung an der Fläche 30 anliegt.

Die vordere Seite 33 der Elektrode 18, die von dem Elektrodenträger 27 abgewandt angeordnet ist kann glatt und ununterbrochen ausgebildet sein. Sie kann bedarfsweise auch gerippt, genoppt, gewellt ausbildet oder mit sonstiger Profilierung versehen sein. Außerdem kann sie mit einer oder mehreren Ausnehmungen versehen sein, in die Elemente, zum Beispiel Keramikteile eingesetzt sein können, um bestimmte Funktionen zu erbringen, wie beispielsweise das Festhalten von Gefäßen oder das Verhindern der Berührung der beiden Elektroden 18, 19 der Branchen 12, 13. Außerdem kann die Elektrode 18 anders als eine ebene Platte geformt sein und zum Beispiel eine schlitzartige Ausnehmung aufweisen, in der zum Beispiel ein Messer beweglich gelagert ist, um koagulierte Gefäße zu durchtrennen.

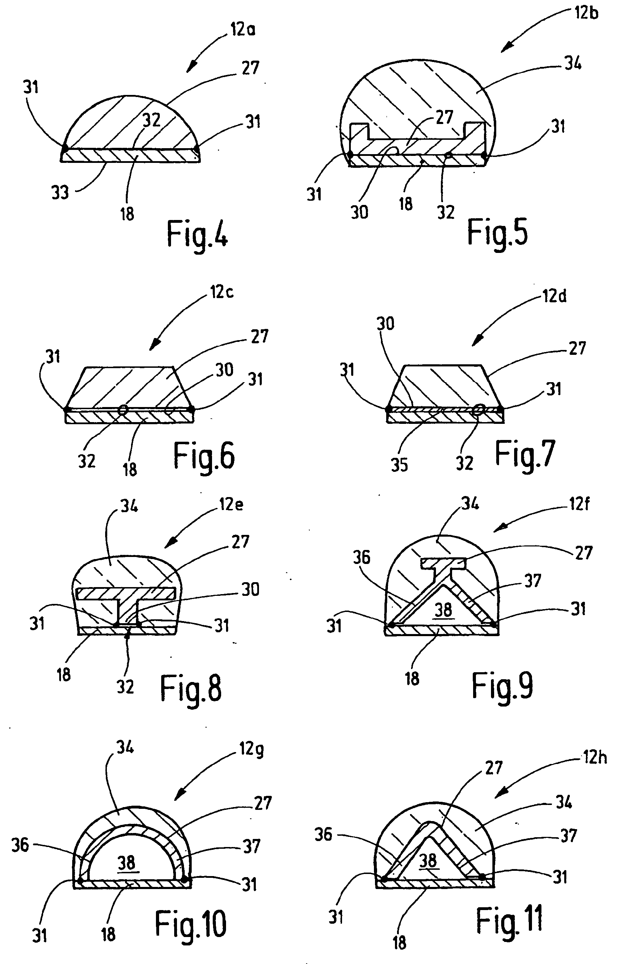

Figur 4 zeigt eine abgewandelte Branche 12a, die im Wesentlichen auf der Branche 12 nach Figur 3 beruht. Wie ersichtlich ist der Elektrodenträger 27 wiederum vollständig aus einem elektrisch leitfähigen Material, zum Beispiel einem Metall oder einer Metalllegierung ausgebildet. Die zur Ausbildung der Verbindungen 31 dienenden Laserschweißpunkte sind in einer Reihe angeordnet, die rings um den Spalt 32 führt.

Figur 5 veranschaulicht eine weiter abgewandelte Branche 12b, die einen Elektrodenträger 27 in Form eines Metallprofils und einen Kunststoffkörper 34 aufweist, in den das Metallprofil des Elektrodenträgers 27 eingebettet ist. Das Metallprofil ist ein U-Profil. Es weist einen Steg auf, an dem die zur Aufnahme der Elektrode 18 dienende Fläche 30 ausgebildet ist. Von dem Steg ausgehend erstrecken sich zwei zueinander parallele Schenkel in den Kunststoffkörper 34 hinein. Die Elektrode 18 ist wiederum über Laserschweißpunkte mit dem Elektrodenträger 27 verbunden. Die Laserschweißpunkte bilden die punktuellen Verbindungen 31, die den Spalt 32 überbrücken und der mechanischen und elektrischen Verbindung dienen. Der Kunststoffkörper 34 umhüllt den Elektrodenträger 27 und erstreckt sich vorzugsweise bis über den Spalt 32, so dass er diesen ringsum abdeckt und abdichtet.

Unabhängig von der Querschnittsform der Branche 12 kann der Spalt 32 kleiner oder aber auch größer ausgebildet sein. Dies gilt für alle Branchenformen. Figur 6 veranschaulicht dazu beispielhaft eine Branche 12c mit einem im Querschnitt trapezförmigen Elektrodenträger 27. Die Elektrode 18 ist von der Fläche 30 etwas beabstandet angeordnet. Die Verbindungen 31 überbrücken den so entstandenen Spalt 32. Der entstandene Luftspalt wirkt isolierend. Wiederum können, wie bei allen vor- und nachbeschriebenen Ausführungsformen, die einander zugewandten Flächen der Elektrode 18 und des Elektrodenträgers 27 glänzend ausgebildet, z.B. poliert sein, um Wärmeübertragung durch Strahlung zu minimieren.

In dem Spalt 32 kann, wie es Figur 7 anhand der Branche 12d zeigt, auch ein oder mehrere Isolierkörper 35 angeordnet sein. Der Isolierkörper 35 kann bspw. durch eine dünne Platte aus Keramik, Glas, Kunststoff, einen Schaum oder sonstigen schlecht wärmeleitenden Material ausgebildet sein. Wiederum dienen Laserschweißpunkte oder ähnliche Löt- oder Schweißpunkte als Verbindungen 31, die den Spalt 32 überbrücken.

Die zur Aufnahme der Elektrode 18 vorgesehen vordere Fläche 30 des Elektrodenträgers 27 muss nicht notwendigerweise die gleiche Kontur haben, wie die Elektrode 18. Figur 8 veranschaulicht dazu eine Branche 12e, bei der der Elektrodenträger 27 als T-Profil ausgebildet ist. Die Fläche 30 zur Aufnahme der Elektrode 18 ist bei dieser Branche 12e an der Stirnfläche des schmalen Stegs des T-Profils ausgebildet. Wiederum ist die Verbindung zwischen der Elektrode 18 und dem Elektrodenträger 27 durch eine Serie von Verbindungen 31, z.B. in Gestalt von Laserschweißpunkten oder auch kurzen Laserschweißnähten, ausgebildet. Zwischen dem Elektrodenträger 27 und der Elektrode 18 kann ein Spalt 32 ohne Abstand oder auch mit einem gewissen Abstand vorgesehen sein. Wiederum ist ein Kunststoffkörper 34 vorgesehen, der an die Rückseite der Elektrode 18 anschließt und den Elektrodenträger 27 einhüllt.

Letzteres ist auch bei der Branche 12f nach Figur 9 gegeben. Der Elektrodenträger 27 weist zwei sich v-artig voneinander weg spreizende Schenkel 36, 37 auf, die über die Verbindungen 31 metallisch stoffschlüssig mit der Elektrode 18 verbunden sind. Der zwischen den Schenkeln 36, 37 und der Elektrode 18 verbleibende Raum 38 kann ein luftgefüllter, ein Kunststoff gefüllter Raum oder ein evakuierter Raum sein oder eine Kombination dieser. Zwischen den Schenkeln 36, 37 und der Elektrode 18 kann ein Spalt von 0 mm bis zu 1 mm vorhanden sein, der von den Verbindungen 31 überbrückt wird. Die Schenkel 36, 37 können gelocht sein, um gasförmige Stoffe oder Festkörperstoffe vorzugsweise wärmeisolierende Stoffe beispielsweise Kunststoff in den Raum 38 einzulassen.

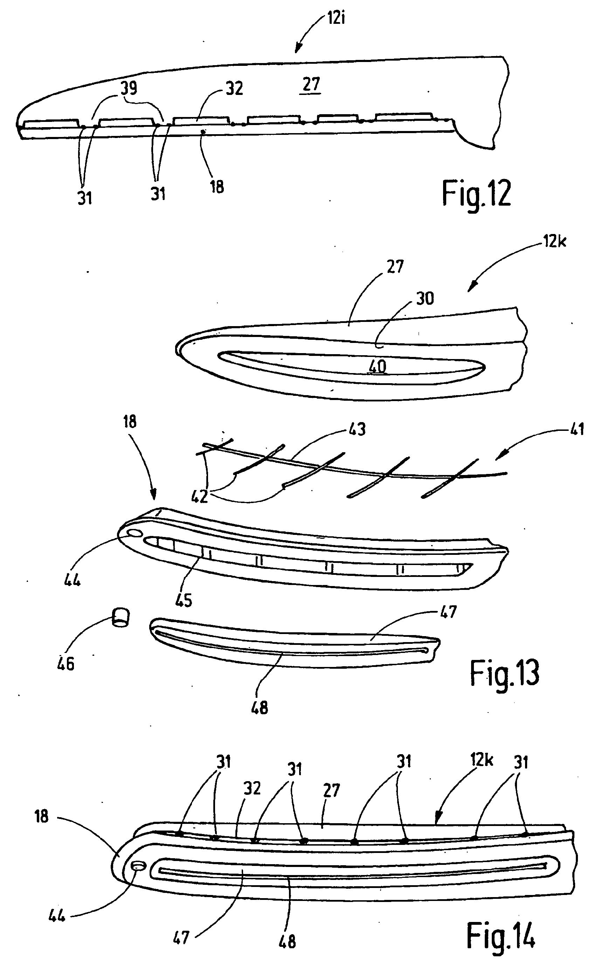

Auch bei der Branche 12g nach Figur 10 oder 12h nach Figur 11 kann zwischen Schenkeln 36, 37 und der Elektrode 18 ein Spalt von 0 mm bis zu 1 mm oder auch größer vorhanden sein, der von den Verbindungen 31 überbrückt wird. Im Unterscheid zu der vorbeschriebenen Branche 12f ist der Elektrodenträger 27 gemäß Figur 10 halbrund und gemäß Figur 11 v-förmig ausgebildet. Wiederum kann der Raum 38 gasgefüllt, leer oder mit Kunststoff gefüllt sein. Zum Einbringen einer Kunststofffüllung kann der Elektrodenträger 27 nicht weiter veranschaulichte Öffnungen aufweisen.

Bei allen vorstehend beschriebenen Ausführungsformen, insbesondere den Ausführungsformen nach Figur 8 bis 11 können die mit den Verbindungen 31 versehenen Kanten des Elektrodenträgers 27 gerade oder auch einer gewünschten Kontur bspw. Zickzack-Kontur, Zinnenkontur, Wellenkontur oder dergleichen folgen. Damit kann zusätzlicher Abstand zwischen z.B. den Schenkeln 36, 37 und der Elektrode 18 geschaffen werden. Die Schenkel 36, 37 nähern sich lediglich an den Verbindungsstellen 31 der Elektrode 18 und halten ansonsten einen größeren Abstand zu dieser ein. Eine derartige Konfiguration ist in Figur 12 anhand der Branche 12i veranschaulicht, die z.B. nach Vorbild einer der Branchen nach Figur 3 bis 7 ausgebildet sein kann. Von dem Elektrodenträger 27 erstrecken sich voneinander beabstandete zinnenartige Vorsprünge 39 zu der Elektrode 18, um den Spalt 32 zu überbrücken. Die Verbindungen 31 sind z.B. durch Laserschweißpunkte oder Laserschweißnähte gebildet, die die Vorsprünge 39 mit der Elektrode 18 verbinden. Die zinnenartigen Vorsprünge 39 können sich entlang des äußeren Rands des Elektrodenträgers 27 erstrecken. Sie können jedoch abweichend auch an anderer Stelle angeordnet sein. Eine solche Zinnenstruktur kann, wie eingangs erwähnt, auch an den Schenkeln 36, 37 vorgesehen sein, wenn die Ausführungsformen nach Figur 8 bis 11 zugrunde gelegt werden.

Figur 13 und 14 veranschaulichen eine weitere Ausführungsform einer Branche 12k. Zu dieser gehört ein Elektrodenträger 27 mit einer zentralen länglichen Ausnehmung 40. Die Ausnehmung 40 unterbricht die Fläche 30. Zwischen der Elektrode 18 und dem Elektrodenträger 27 ist ein Abstandshalter 41 angeordnet, der bspw. durch mehrere Drähte 42 gebildet wird, die untereinander durch einen Verbindungsdraht 43 verbunden sein können. Die Drähte 42 bilden mit dem Verbindungsdraht 43 eine Leiterstruktur, eine Gitterstruktur oder dergleichen. Die Elektrode 18 weist eine oder mehrere Öffnungen 44, 45 auf. Die vorzugsweise zylindrische Öffnung 44 dient z.B. der Aufnahme eines Keramikstifts 46, der einen Kurzschluss zwischen einander gegenüber liegenden Elektroden verhindern soll. Die Öffnung 45 dient bspw. zur Aufnahme eines aus Kunststoff oder Keramik bestehenden länglichen Einlegers 47, der z.B. zum Festklemmen von Gefäßen zwischen den Elektroden 18, 19 dient und dazu eine mittige erhabene Rippe 48 aufweist. In zusammengebautem Zustand stehen die stirnseitigen Enden der Drähte 42 des Abstandshalters 41 am Rand des Spalts 32. Mit einem Laserstrahl oder einem anderen geeigneten Mittel können die Enden der Drähte 42 zu Schweißpunkten umgeformt werden, die eine feste punktuelle metallische Verbindung zwischen der Elektrode 18 und dem Elektrodenträger 27 schaffen, wie es Figur 14 zeigt. Damit können die Drähte 42 nicht nur als Abstandshalter dienen, sondern zugleich Schweißzusatzwerkstoff zum Herstellen der Verbindungen 31 bereitstellen.

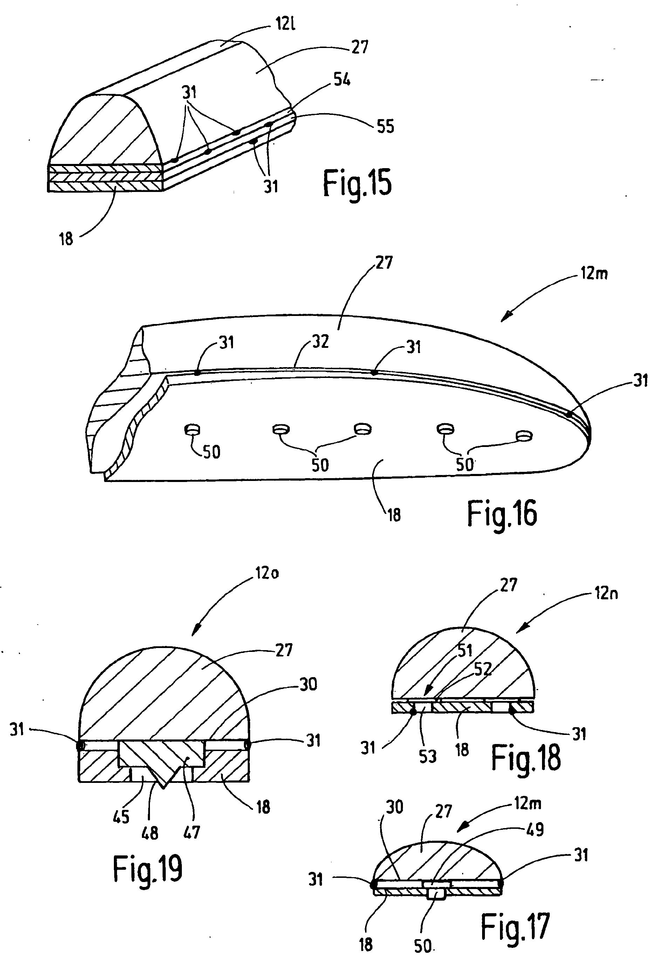

Eine weitere Ausführungsform der Branche 121 ist in Figur 15 veranschaulicht. Zwischen der Elektrode 18 und dem Elektrodenträger 27 ist mindestens ein weiteres metallisches Element angeordnet. Zum Beispiel können zwischen der Elektrode 18 und dem Elektrodenträger 27 ein, zwei oder mehrere dünne Platten 54, 55, Ringe, Netze oder dergleichen angeordnet sein. Somit werden zwischen der Elektrode 18 und dem Elektrodenträger 27 mehrere Spalte ausgebildet, die jeweils als Wärmebarrieren wirken. Die Verbindungspunkte 31 sind jeweils spaltüberbrückend außen vorgesehen und sichern die Elektrode 18 letztendlich metallisch stoffschlüssig an dem Elektrodenträger 27.

Es sind weitere Abwandlungen möglich. Zum Beispiel zeigt Figur 16 eine Branche 12m, deren Elektrodenträger 27 schematisch nach dem Vorbild von Figur 3 ausgebildet ist. Es kann jedoch auch jeder andere Elektrodenträger nach Figur 4 bis 7, sowie Figur 9 bis 12 Anwendung finden.

Bei dem Ausführungsbeispiel nach Figur 16 sind zwischen dem Elektrodenträger 27 und der Elektrode 18 Abstandshalter 49 angeordnet, wie sie aus Figur 17 ersichtlich sind. Diese Abstandshalter 49 sind bspw. durch Keramikscheiben gebildet. Von diesen können sich zylinderartige Fortsätze 50 durch in der Elektrode 18 ausgebildete Öffnungen erstrecken. Die Fortsätze 50 können z.B. dazu dienen, ein Verrutschen von zwischen den Elektroden 18, 19 aufgenommenen Gefäßen sowie eine Berührung der gegenüber liegenden Elektroden 18, 19 untereinander zu verhindern. Die Abstandshalter 49 können ein- oder doppelreihig oder auf sonstige Weise angeordnet sein. Die Fortsätze 50 können mit den Keramikscheiben einstückig, nahtlos ausgebildet sein. Alternativ können die Abstandshalter 49 durch Ringe aus Metall, Kunststoff oder Keramik gebildet sein, die auf Stiften 50 aus Metall, Keramik oder Kunststoff sitzen.

Der zwischen der Elektrode 18 und dem Elektrodenträger 27 verbleibende Spalt ist durch Schweißpunkte überbrückt, die vorzugsweise an dem äußeren Rand des Spalts 32 angeordnet sind. Die Fläche 30 kann, wie dargestellt, eben oder an ihrem Rand auch zu der Elektrode 18 hin gewölbt ausgebildet sein, um die Weite des Spalts 32 am Rand der Elektrode 18 zu vermindern. Auch kann der Rand der Fläche 30 mit Vorsprüngen aller Art, z.B. Zinnen nach Figur 12 versehen sein.

Anstelle der keramischen Abstandshalter 49 können auch metallische Abstandselemente 51 vorgesehen sein, wie es Figur 18 anhand der Branche 12n zeigt. Die Abstandselemente 51 können ein- oder doppelreihig oder auf sonstige Weise angeordnet sein. Sie können wiederum aus einer Scheibe 52 mit einem sich anschließenden Fortsatz 53 gebildet sein. Die Abstandselemente 51 können bspw. einstückig mit dem Elektrodenträger 27 verbunden oder stumpf an diesem angeschweißt sein. Sie können auch durch kurze Bolzen gebildet sein, die die Scheibe 52 als Unterlegscheibe tragen und in Gewindebohrungen des Elektrodenträgers 27 eingeschraubt oder an dessen Fläche 30 angeschweißt sind. Die Fortsätze 53 können sich durch Öffnungen der Elektrode 18 erstrecken und an ihrem stirnseitigen Rand mit der Elektrode 18 verschweißt sein. Die Verbindungspunkte 31 sind damit nicht am Rand der Elektrode 18 sondern am Rand von Öffnungen der Elektrode 18 vorgesehen. Auch dieses Prinzip ist auf nahezu alle vorstehend beschriebenen Ausführungsformen übertragbar, insbesondere auf die Ausführungsform nach Figur 4 bis 12. Die Ausführungsform nach Figur 18 kann auch mit der Ausführungsform nach Figur 16 kombiniert werden, indem bei letzterer zusätzlich zu den keramischen Abstandshaltern 49 stifte oder Bolzen vorgesehen werden, die den Fortsätzen 53 entsprechen und sich durch die Elektrode 18 erstrecken um mit dieser verschweißt zu sein.

Eine weitere Ausführungsform der Erfindung zeigt Figur 19. Die hier dargestellte Branche 12o weist einen Elektrodenträger 27 auf, der dem Elektrodenträger 27 nach Figur 4 entspricht. Es kann aber auch ein Elektrodenträger, z.B. nach Figur 5 bis 7, Figur 12 oder Figur 15 vorgesehen sein. In der Elektrode 18 ist ähnlich wie bei dem Ausführungsbeispiel 13 und 14 wiederum eine längliche Öffnung 45 vorgesehen, in der der Einleger 47 angeordnet ist. Die Öffnung 45 ist gestuft ausgebildet. Der Einleger 47 füllt den weiteren (oberhalb der Stufe stehenden) Teil der Öffnung 45 aus und ruht an dem Elektrodenträger 27 bzw. seiner Fläche 30. Der Einleger 47 ist etwas dicker als der Abstand zwischen der Stufe der Elektrode 18 und der Fläche 30, so dass der Einleger 27 als Abstandshalter wirkt. Die Verbindungspunkte 31 stellen wiederum eine feste Verbindung zwischen der Elektrode 18 und dem Elektrodenträger 27 her.

In Betrieb wird zwischen den Branchen 12, 13 ein Gefäß gefasst und zusammengedrückt. Durch Bestromung der Elektroden 18, 19 bspw. mit HF-Strom wird das Gewebe erwärmt und es tritt die gewünschte Koagulation auf. Die am Gewebe entstehende Wärme überträgt sich, zumindest teilweise, auf die Elektroden 18, 19, wodurch sich diese mit erwärmen. Die Wärme kann jedoch nicht ohne weiteres in den Elektrodenträger 27 abfließen. Somit kühlt der Elektrodenkörper 27 die Elektrode 18, 19 wenig, so dass sich die gewünschte Wärmewirkung in dem Gefäß ungehindert entfalten kann. Der Elektrodenträger 27 nimmt dabei sehr wenig Wärme auf, so dass sich die Branchen 12, 13 an ihren Außenseiten kaum nennenswert erwärmen. Die thermische Isolation durch den Kunststoffkörper 34 oder eine Kunststoffbeschichtung verhindert Wärmefluss aus der Branche 12, 13 in das biologische Gewebe zusätzlich. Eine Gewebeschädigung wird dadurch vermieden.

Das erfindungsgemäße Gewebefusionsinstrument 10 weist Elektroden 18, 19 auf, die nur punktuell mit ihrem Elektrodenträger 27 elektrisch leitend und somit stoffschlüssig wärmeleitend verbunden sind. Die Wärmeübertragung von der Elektrode 18, 19 auf den jeweiligen Elektrodenträger 27 ist dadurch eingeschränkt. Dies kommt einerseits dem gewünschten chirurgischen Ergebnis zugute und verhindert andererseits eine parasitäre Erwärmung des chirurgischen Instruments und eine damit einhergehende Schädigung des umgebenden Gewebes.

### Bezugszeichenliste:

- 10: Gewebefusionsinstrument
- 11: Gewebefusionszange
- 12: erste Branche (Alternativen: 12a bis 12o)
- 13: zweite Branche
- 14: Scharniergelenk
- 15, 16: Handgriff
- 17: Zuleitung
- 18, 19: Elektrode
- 20: Zuleitung
- 21: laproskopisches Instrument
- 22: Gehäuse
- 23: Griff
- 24: Schaft
- 25: Werkzeug
- 26: beweglicher Teil des Griffs 23
- 27: Elektrodenträger
- 28: Ende
- 29: Rücken
- 30: Fläche
- 31: Verbindungen
- 32: Spalt
- 33: Fläche
- 34: Kunststoffkörper
- 35: Isolierkörper
- 36, 37: Schenkel
- 38: Raum
- 39: Vorsprünge
- 40: Ausnehmung
- 41: Abstandshalter
- 42: Drähte
- 43: Verbindungsdraht
- 44, 45: Öffnungen
- 46: Stift
- 47: Einleger
- 48: Rippe
- 49: Abstandshalter
- 50: Fortsatz
- 51: Abstandselement
- 52: Scheibe
- 53: Fortsatz
- 54, 55: Plättchen

## Patentansprüche

1. Gewebefusionszange (11),
mit einer ersten Branche (12) und einer zweiten Branche (13), die untereinander gelenkig verbunden sind, um aufeinander zu und voneinander weg bewegt werden zu können, und
mit an den Branchen (12, 13) vorhandenen, als ebene oder gekrümmte Platte ausgebildeten Elektroden (18, 19) zur Koagulation eines zwischen den Branchen (12, 13) erfassten Gefäßes,
wobei die Branche (12, 13) einen Elektrodenträger (27) aufweist, der mit einer elektrischen Zuleitung (17) verbunden oder verbindbar ist,
wobei die Elektrode (18) an einer Seite des Elektrodenträgers (27) angeordnet ist,
**dadurch gekennzeichnet,**
**dass** die Elektrode (18) über eine oder mehrere punktuelle Verbindungen (31) elektrisch mit dem Elektrodenträger (27) verbunden
und **dass** zwischen der Elektrode (18) und dem Elektrodenträger (27) ein Spalt (32) ausgebildet ist.

2. Gewebefusionszange nach Anspruch 1, **dadurch gekennzeichnet, dass** die punktuelle elektrische Verbindung (31) durch einen oder mehrere Klebe-, Löt- oder Schweißpunkte oder eine oder mehrere Schweißnähte gebildet ist.

3. Gewebefusionszange nach Anspruch 1, **dadurch gekennzeichnet, dass** die Elektrode (18) durch die punktuelle elektrische Verbindung (31) mechanisch an dem Elektrodenträger (27) befestigt ist.

4. Gewebefusionszange nach Anspruch 1, **dadurch gekennzeichnet, dass** die Elektrode (18) mit einer oder mehreren Öffnungen (44, 45) versehen ist.

5. Gewebefusionszange nach Anspruch 4, **dadurch gekennzeichnet, dass** in zumindest einer der Öffnungen (44, 45) ein elektrischer Isolator (46, 47) angeordnet ist.

6. Gewebefusionszange nach Anspruch 5, **dadurch gekennzeichnet, dass** sich der elektrische Isolator (46, 47) zwischen dem Elektrodenträger (27) und der Elektrode (18) erstreckt und in oder durch die Öffnung (44, 45) ragt.

7. Gewebefusionszange nach Anspruch 1, **dadurch gekennzeichnet, dass** der Spalt (32) durch Vorsprünge (39) des Elektrodenträgers (27) und/oder der Elektrode (18) überbrückt ist.

8. Gewebefusionszange nach Anspruch 1, **dadurch gekennzeichnet, dass** in dem Spalt (32) ein Abstandshalter (41, 47, 49, 52) angeordnet ist.

9. Gewebefusionszange nach Anspruch 8, **dadurch gekennzeichnet, dass** der Abstandshalter (41) aus metallischem oder nichtmetallischem Werkstoff oder einer Kombination beider besteht.

10. Gewebefusionszange nach Anspruch 8, **dadurch gekennzeichnet, dass** der Abstandshalter (49) aus einem Kunststoff oder Keramik besteht.

11. Gewebefusionszange nach Anspruch 1, **dadurch gekennzeichnet, dass** zwischen der Elektrode (18) und dem Elektrodenträger (27) eine oder mehrere Zwischenplatten (54, 55) angeordnet sind.

12. Gewebefusionszange nach Anspruch 1, **dadurch gekennzeichnet, dass** der Elektrodenträger (27) an seiner von der Elektrode (18) abgewandten Seite mit elektrisch isolierendem Material (34) versehen ist.

13. Gewebefusionszange nach Anspruch 12, **dadurch gekennzeichnet, dass** sich das elektrisch isolierende Material (34) bis zu der Elektrode (18) erstreckt.

## Claims

1. Tissue fusion forceps (11),
with a first arm (12) and a second arm (13) which are connected to one another in an articulated manner such as to enable them to be moved towards and away from one another, and
with electrodes (18, 19), which are present on the arms (12, 13) and are designed as a flat or curved plate, for the coagulation of a vessel that is clamped between the arms (12, 13),
wherein the arm (12, 13) has an electrode holder (27) that is or can be connected to an electrical power supply (17),
wherein the electrode (18) is arranged on one side of the electrode holder (27),
**characterised in that**
the electrode (18) is electrically connected to the electrode holder (27) via one or more puncitorm connections (31)
and that a gap (32) is formed between the electrode (18) and the electrode holder (27).

2. Tissue fusion forceps according to claim 1, **characterised in that** the punctiform electrical connection (31) is formed by one or more adhesive, solder or weld spots or one or more weld seams.

3. Tissue fusion forceps according to claim 1, **characterised in that** the electrode (18) is affixed mechanically to the electrode holder (27) by means of the punctiform electrical connection (31).

4. Tissue fusion forceps according to claim 1, **characterised in that** the electrode (18) is equipped with one or more openings (44,45).

5. Tissue fusion forceps according to claim 4, **characterised in that** an electrical insulator (46, 47) is arranged in at least one of the openings (44, 45).

6. Tissue fusion forceps according to claim 5, **characterised in that** the electrical insulator (46, 47) extends between the electrode holder (27) and the electrode (18) and projects into or through the opening (44,45).

7. Tissue fusion forceps according to claim 1, **characterised in that** the gap (32) is bridged by projections (39) of the electrode holder (27) and/or of the electrode (18).

8. Tissue fusion forceps according to claim 1, **characterised in that** arranged in the gap (32) is a spacer (41, 47, 49, 52).

9. Tissue fusion forceps according to claim 8, **characterised in that** the spacer (41) comprises metallic or non-metallic material or a combination of the two.

10. Tissue fusion forceps according to claim 8, **characterised in that** the spacer (49) comprises a plastic or ceramic.

11. Tissue fusion forceps according to claim 1, **characterised in that** arranged between the electrode (18) and the electrode holder (27) are one or more intermediate plates (54, 55).

12. Tissue fusion forceps according to claim 1, **characterised in that** the electrode holder (27) is equipped with electrically insulating material (34) on its side facing away from the electrode (18).

13. Tissue fusion forceps according to claim 12, **characterised in that** the electrically insulating material (34) extends as far as the electrode (18).

## Revendications

1. Pince de fusion tissulaire (11),
comprenant un premier mors (12) et un deuxième mors (13) qui sont reliés entre eux de façon articulée, afin de pouvoir être rapprochés et éloignés l'un de l'autre, et
comprenant des électrodes (18, 19) prévues sur les mors (12, 13) et réalisées sous forme de plaque plane ou incurvée, destinées à la coagulation d'un vaisseau saisi entre les mors (12, 13),
le mors (12, 13) présentant un support d'électrode (27) qui est relié ou peut être relié à une ligne d'alimentation électrique (17),
l'électrode (18) étant disposée sur un côté du support d'électrode (27),
**caractérisée en ce que**
l'électrode (18) est reliée électriquement par une ou plusieurs liaisons (31) ponctuelles au support d'électrode (27),
et **en ce qu'**un interstice (32) est formé entre l'électrode (18) et le support d'électrode (27).

2. Pince de fusion tissulaire selon la revendication 1; **caractérisée en ce que** la liaison électrique (31) ponctuelle est constituée d'un ou plusieurs points de collage, de brasage ou de soudage ou d'une ou plusieurs soudures.

3. Pince de fusion tissulaire selon la revendication 1; **caractérisée en ce que** l'électrode (18) est fixée mécaniquement au support d'électrode (27) par la liaison électrique (31) ponctuelle.

4. Pince de fusion tissulaire selon la revendication 1; **caractérisée en ce que** l'électrode (18) est pourvue d'une ou plusieurs ouvertures (44, 45).

5. Pince de fusion tissulaire selon la revendication 4; **caractérisée en ce que** dans au moins une des ouvertures (44, 45), est disposé un isolateur électrique (46, 47).

6. Pince de fusion tissulaire selon la revendication 5; **caractérisée en ce que** l'isolateur électrique (46, 47) s'étend entre le support d'électrode (27) et l'électrode (18) et avance dans l'ouverture (44, 45) ou la traverse.

7. Pince de fusion tissulaire selon la revendication 1; **caractérisée en ce que** l'interstice (32) est traversé par des saillies (39) du support d'électrode (27) et/ou de l'électrode (18).

8. Pince de fusion tissulaire selon la revendication 1, **caractérisée en ce qu'**un espaceur (41, 47, 49, 52) est disposé dans l'interstice (32).

9. Pince de fusion tissulaire selon la revendication 8, **caractérisée en ce que** l'espaceur (41) est constitué d'un matériau métallique ou non métallique ou d'une combinaison des deux.

10. Pince de fusion tissulaire selon la revendication 8, **caractérisée en ce que** l'espaceur (49) est constitué d'une matière plastique ou de céramique.

11. Pince de fusion tissulaire selon la revendication 1, **caractérisée en ce qu'**une ou plusieurs plaques intermédiaires (54, 55) sont disposées entre l'électrode (18) et le support d'électrode (27).

12. Pince de fusion tissulaire selon la revendication 1, **caractérisée en ce que** sur sa face éloignée de l'électrode (18), le support d'électrode (27) est pourvu d'un matériau (34) électriquement isolant.

13. Pince de fusion tissulaire selon la revendication 12, **caractérisée en ce que** le matériau (34) électriquement isolant s'étend jusqu'à l'électrode (18).
